# EUROPEAN PATENT APPLICATION

(11) **EP 3 970 755 A1**
(43) Date of publication of application: **23.03.2022**
(21) Application number: 21184779.3
(22) Date of filing: 09.07.2021

(54) **SURFACE DECONTAMINATION APPARATUS AND METHOD**

(30) Priority: 30.07.2020 US 202063058788 P
(71) Applicant: Luminator Holding, L.P., Plano, TX 75074 (US)
(72) Inventor: Malcherek, Werner, Plano, 75074 (US); Goins, Kirk, Plano, 75074 (US)
(74) Representative: Pöhner, Wilfried Anton

(57) **Abstract**

An apparatus (100) for decontaminating a surface is provided. Embodiments include a first laser (106) and a second laser (108) with the first laser (106) emitting a first beam of light and the second laser (108) emitting a second beam of light. The first laser (106) is an IR laser with the first beam of light comprising wavelengths between 700 nanometers and 1 millimeter. The second laser (108) is an UV laser with the second beam of light comprising wavelengths between 10 nanometers and 450 nanometers. A method for decontaminating a surface is also provided. Embodiments of the method include aiming an IR laser at a surface and energizing the IR laser to decontaminate the surface. The method further includes aiming an UV laser at the surface and energizing the UV laser to decontaminate the surface. The method further includes scanning the IR and UV lasers across the surface when they are energized.

## Description

### TECHNICAL FIELD

This disclosure relates to decontamination and more particularly, to surface decontamination.

### BACKGROUND

Ultraviolet (UV) radiation has been employed for disinfection and decontamination of surfaces, air, and liquids. Generally, UV lamps are used to disinfect surfaces from bacteria, viruses, and fungi by generating UV rays. As the UV lamps are in the form of a lamp, the UV lamps may be appropriately used through manual manipulation when necessary. Moreover, as UV rays generated by the UV lamps are hardly changed, the UV rays continuously maintain a same disinfection power.

The UV lamps generate UV rays having various wavelengths according to a material used therein. For example, a UV lamp may generate UV-A (wavelength of 400 nm to 315 nm), UV-B (wavelength of 15 nm to 280 nm), or UV-C (wavelength of 280 nm to 110 nm). When the UV-C is irradiated to a DNA of the bacteria and fungus, the DNA of the bacteria and fungus is damaged and destroyed. That is, the UV rays damage a DNA of a living organism and have an effective disinfecting power with respect to various bacteria. However, the disinfection is not as effective if an obstacle is between the UV rays and the bacteria, viruses, and fungi. Even obstacles such as dust and debris can temper the effectiveness of the UV rays. In some applications, a target requires cleaning before UV rays can be used effectively. Further, the disinfection may not be as effective if the UV rays are not directly orthogonal to a surface to be disinfected.

UV lamps have been made with mercury or mercury amalgams. These UV lamps can be bulky and consume large amounts of energy. In addition, in order to exhibit a disinfection power to destroy various bacteria, UV rays generated by UV disinfection lamps need to uniformly irradiate a surface for a predetermined period of time. Thus, another issue is to irradiate uniform UV rays on a surface. Further, even when a large-sized UV lamp is installed, a uniform plane may not be uniformly disinfected. Moreover, particles on the surface may block some of the UV radiation from reaching the surface in order to disinfect the surface.

UV lamps are commonly used in stationary applications, such as in rooms within buildings. However, harmful bacteria, viruses, and fungi exist in many locations where traditional stationary UV lamps would are not feasible. Additionally, traditional stationary UV lamps cannot move so that the UV light reaches substantially all parts of a surface.

### SUMMARY

In one aspect, this disclosure is directed to an apparatus for decontaminating surfaces comprising a body comprising one or more motors for maneuvering the body and a power source supported by the body. The apparatus further comprises a first laser supported by the body and powered by the power source. The first laser is configured to emit a first beam of light which comprises wavelengths between 700 nanometers and 1 millimeter. The apparatus further comprises a second laser supported by the body and power by the power source. The second laser is configured to emit a second beam of light which comprises wavelengths between 10 nanometers and 450 nanometers. The first laser and the second laser are able to remove contaminants from a surface by emitting the first beam of light and the second beam of light onto the surface.

In a second aspect, this disclosure is directed to a method of decontaminating a surface comprising determining if a surface is contaminated with debris. The method further comprises measuring a surface area and a distance to the surface and aiming an IR laser at the surface. An amount of IR energy to be used is defined. The method further comprises scanning the IR laser across the surface with the IR laser configured to emit a first beam of light. The first beam of light comprises wavelengths between 700 nanometers and 1 millimeter. The method further comprises aiming a UV laser at the surface and defining an amount of UV energy to be used. The method further comprises scanning the UV laser across the surface with the UV laser configured to emit a second beam of light. The second beam of light comprises wavelengths between 10 nanometers and 450 nanometers. The scanning of the IR laser and the UV laser decontaminating the surface.

In a third aspect, this disclosure is directed to a method of decontaminating a surface comprising maneuvering a decontamination apparatus to a position, the position being a distance from a surface. The decontamination apparatus comprises a first laser and as second laser wherein the first laser is configured to emit a first beam of light. The first beam of light comprises wavelengths between 700 nanometers and 1 millimeter. The second laser is configured to emit a second beam of light with the second beam of light comprising wavelengths between 10 nanometers and 450 nanometers. The method further includes measuring a surface area and/or the distance to the surface and aiming the first laser and/or the second laser at the surface. The method further comprises energizing the first and/or second laser such that it emits a beam of light toward the surface. The method still further comprises scanning the first laser and/or the second laser across the surface, the scanning of the energized first and/or second laser decontaminating the surface.

In a fourth aspect, this disclosure is directed to an apparatus for decontaminating a surface comprising a body with one or more supports for supporting the body above a ground surface. The apparatus further comprises a power source supported by the body and a first laser supported by the body. The first laser is powered by the power source and the first laser is configured to emit a first beam of light. The first beam of light comprises wavelengths between 700 nanometers and 1 millimeter. The apparatus further comprises a second laser supported by the body and powered by the power source. The second laser is configured to emit a second beam of light with the second beam of light comprising wavelengths between 10 nanometers and 450 nanometers. The first laser is configured to direct the first beam of light toward a surface and the second laser is configured to direct the second beam of light toward the surface, the first beam of light and the second beam of light for decontaminating the surface.

In a fifth aspect, this disclosure is directed to an apparatus for decontaminating a surface comprising a body and a first laser supported by the body. The first laser is configured to emit a first beam of light with the first beam of light comprising wavelengths between 700 nanometers and 1 millimeter. The apparatus further comprises a second laser supported by the body and configured to emit a second beam of light. The second beam of light comprises wavelengths between 10 nanometers and 450 nanometers. The first laser and the second laser are able to remove contaminants from the surface by emitting the first beam of light and the second beam of light onto the surface.

In a sixth aspect, this disclosure is directed to a method for decontaminating a surface comprising aiming the IR laser at a surface. The IR laser configured to emit a first beam of light, the first beam of light comprising wavelengths between 700 nanometers and 1 millimeter. The method further comprises energizing the IR laser such that the first beam of light hits the surface. The method further comprises aiming a UV laser at the surface with the UV laser configured to emit a second beam of light. The second beam of light comprises wavelengths between 10 nanometers and 450 nanometers. The method further comprises energizing the UV laser such that the second beam of light hits the surface. The first beam of light and the second beam of light decontaminate the surface.

### BRIEF DESCRIPTION OF DRAWINGS

FIG. 1A is a perspective view of an aerial vehicle with decontamination abilities according to an aspect of the present disclosure.
FIG. 1B is a perspective view of a moveable apparatus with decontamination abilities according to an aspect of the present disclosure.
FIG. 1C is a perspective view of a ground vehicle with decontamination abilities according to an aspect of the present disclosure.
FIG. 2 is a perspective view of an exemplary aerial vehicle with decontamination abilities according to an aspect of the present disclosure.
FIG. 3 is a schematic view of an example laser with scanning mirrors according to an aspect of the present disclosure.
FIG. 4A is a schematic view of an example apparatus using an IR laser to decontaminate a surface according to an aspect of the present disclosure.
FIG. 5A is a schematic view of an example apparatus using an IR laser to decontaminate a surface by ablating a portion of a surface according to an aspect of the present disclosure.
FIG. 5B is a schematic view of the example apparatus of FIG. 5A using an UV laser to decontaminate a surface according to an aspect of the present disclosure.
FIG. 6 is a perspective view of an example aerial vehicle with an IR laser and UV laser decontaminating an indoor area according to an aspect of the present disclosure.
FIG. 7 is a perspective view of an example aerial vehicle with an IR laser and a UV laser decontaminating an outdoor area according to an aspect of the present disclosure.
FIG. 8 is an example flow diagram of decontaminating a surface according to an aspect of the present disclosure.

### DETAILED DESCRIPTION

The following detailed description is exemplary in nature and is not intended to limit the scope, applicability, or configuration of the invention in any way. Rather, the following description provides some practical illustrations for implementing examples of the present invention. Examples of constructions, materials, dimensions, and manufacturing processes are provided for selected elements, and all other elements employ that which is known to those of ordinary skill in the field of the invention. Those skilled in the art will recognize that many of the noted examples have a variety of suitable alternatives.

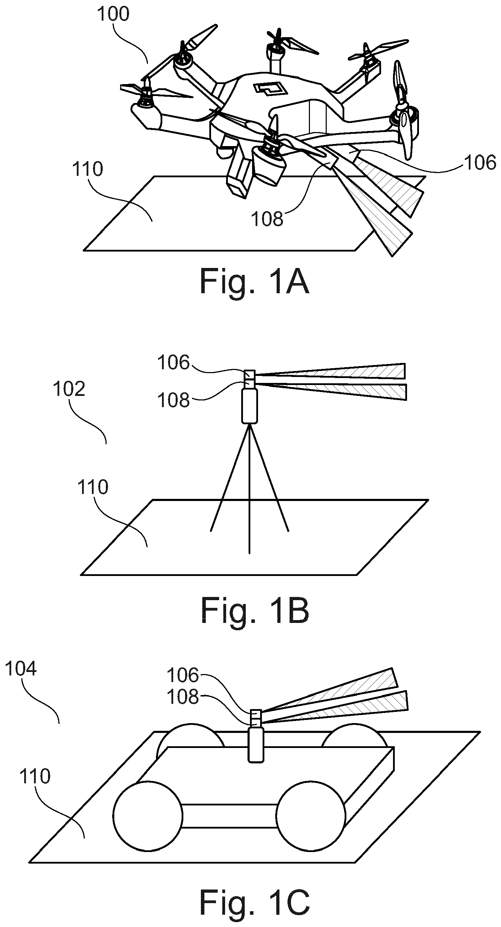

FIG. 1A is a perspective view of an aerial vehicle 100 with decontamination capabilities according to an aspect of the present disclosure. FIG. 1B is a perspective view of a moveable stand 102 with supports having decontamination abilities according to an aspect of the present disclosure. FIG. 1C is a perspective view of a ground vehicle 104 with decontamination abilities according to an aspect of the present disclosure. Decontamination capabilities can include disinfection, sanitization, sterilization, ablation, or other methods which remove contaminants from a surface. Contaminants can include micro-organisms, hazardous materials, chemicals, radioactive substances, and infectious diseases, however, a person having ordinary skill in the art will appreciate other contaminants are contemplated.

The examples of FIG. 1A - 1C include a first laser 106 and a second laser 108 which can decontaminate a surface as described further herein. FIG. 1A is an example of is an example of an aerial vehicle 100 which can maneuver above a ground surface such as in the atmosphere. FIG. 1B is an example of a moveable stand 102 which is moveable about a ground surface 110. Once the moveable stand 102 has been moved to a desired location, the moveable stand 102 can remain stationary while using the first laser 106 and the second laser 108. FIG. 1C is an example of a ground vehicle 104 which can maneuver about a ground surface 110. One of ordinary skill will appreciate that other objects to which the first laser 106 and second laser 108 can be attached to are contemplated and that the present disclosure is not limited to vehicles, or moveable apparatuses.

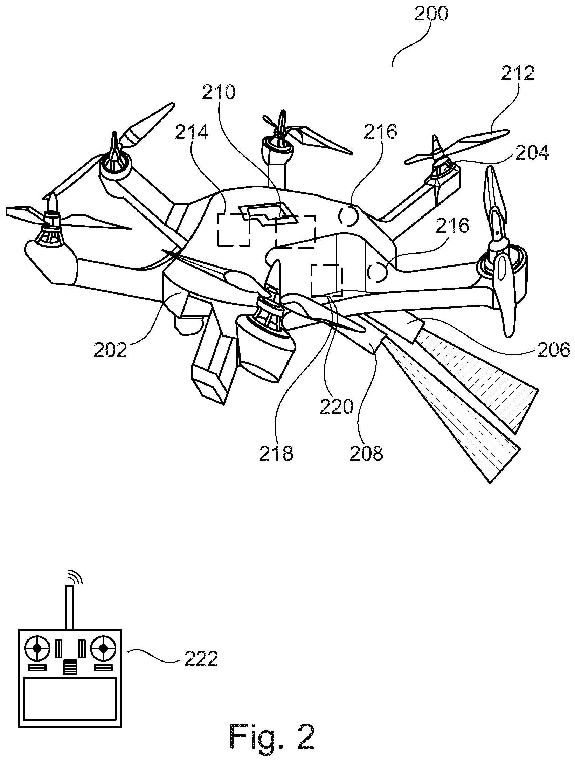

FIG. 2 is a perspective view of an exemplary aerial vehicle 200 with decontamination capabilities according to the present disclosure. The aerial vehicle 200 includes a vehicle body 202, motors 204 connected to the vehicle body 202, and a power source 210 supported by the vehicle body 204 which can provide power to the motors 204. The aerial vehicle 200 further includes a first laser 206 and a second laser 208. In some examples, the motors 204 are electric motors. The motors 204 are connected to the rotor blades 212 and can cause the rotor blades 212 to rotate In the example of FIG. 2, when the rotor blades 212 rotate, they provide a force substantially orthogonal to the horizontal plane in which they rotate (e.g. lift). This force (e.g. lift) can be used to counteract the gravitational force acting on the aerial vehicle 200 and can allow the aerial vehicle 200 to fly.

Continuing with FIG. 2, the motors 204 are in electronic communication with a controller 214 (e.g. processor, ASIC, FPGA). Controller 214 can control the motors 204 and rotor blades 212 and various aspects of the motors 204 and rotor blades 212. For example, the controller 214 can change the rpm of any single motor 204 such that one or more rotor blades 212 rotate at a different rpm. In some examples, the controller 214 can change the rpm of multiple motors at substantially the same time such that one or more rotor blades rotate at different rpms. In some embodiments, the controller 214 can change the rpm of one rotor blade to a first rpm while changing one or more other rotor blades to a second rpm different than the first rpm. In the example of FIG. 2, the controller 214 can change the rpm of substantially all the rotor blades 212 at the same time such that the vehicle adjusts it altitude. Additionally, the controller 214 can change the rpm of a single rotor blade such that the vehicle adjusts its pitch or roll. By adjusting the pitch or roll, the controller can also control the direction the vehicle is flying (e.g. laterally and/or longitudinally). Furthermore, the controller 214 can change the rpm of substantially all the rotor blades rotating clockwise or counterclockwise such that the vehicle adjusts its yaw. In some examples, the controller 214 can change the rpm of one more rotor blades independently of any other one or more rotor blades. In some examples, by changing the rpms of individual rotor blades, the controller can control the pitch, roll, yaw simultaneously such that the vehicle can be maneuvered (e.g. flown) with 6 degrees of freedom, or in some example, omnidirectionally.

In some examples, the controller 214 can be preprogramed to execute certain maneuvers. The maneuvers can be stored in a computer readable medium such that a processor can deliver the maneuvers to the controller from the computer readable medium. The controller 214 can then execute the maneuvers sent to it such that the vehicle follows a specific pattern of maneuvers. In some examples, the area which is to be decontaminated is scanned prior to decontamination. In some such examples, the vehicle is sent instructions such that it can navigate the area using the scanned information. In some examples, the controller 214 is sent instructions such that it can maneuver the vehicle in a predetermined path.

In the example of FIG. 2, the vehicle 200 includes video cameras 216 a global positioning system (GPS) sensor 218, and an altitude sensor 220 in addition to controller 214. The video cameras 216 can relay visual information to the controller 214 and/or to a processor connected to the controller ##. In some examples, the visual information can be used by the processor to determine where the vehicle is within 3-dimensional space. This can then allow the controller to avoid obstacles when maneuvering the vehicle. Additionally, in some examples, the visual information can be used by the processor to decide to where to maneuver the vehicle. For example, the visual information could be used to maneuver the vehicle into a certain position relative to a surface or other object. In some examples, the visual information can be recorded, sent wirelessly (e.g. to a second controller), and/or used to identify specific objects/surfaces as described further herein. In some examples, object recognition is used. Object recognition using video cameras 216 can be advantageous as the vehicle can recognize the objects which need to be decontaminated. In FIG. 2, the GPS sensor 218 can also provide the controller 214 with location information such that the controller can determine where the vehicle is within 3-dimensional space. The altitude sensor 220 (e.g. a barometer) of FIG. 2 can be used by the controller to determine the altitude of the vehicle. In some examples, the GPS system 218 can work in tandem with the altitude sensor 220 and controller 214 so that the controller 214 can better determine where the vehicle is within 3-dimensional space. In still further examples, the video cameras 216, GPS sensor 218, and altitude sensor 220 work with the controller to provide an accurate location of the vehicle in 3-dimensional space so that the controller can maneuver the vehicle precisely within 3-dimensional space.

In some embodiments, the vehicle can include other navigational elements such as an ultrasonic pulse system. The ultrasonic pulse system can be used to help the vehicle navigate by sending and receiving ultrasonic pulses to determine distances to objects. In some examples, the vehicle can use a radar sensor system to help navigate by sending and receiving radar pulses to determine its position relative to other objects. Further, in some examples, a laser sensor can be used to help the vehicle navigate by sending and receiving laser light to objects in an area around the vehicle. It will be appreciated that other sensors alone or in combination with each other and/or with the sensors described above, which aid in the navigation of the vehicle are contemplated.

In some examples, the controller can be wirelessly connected to a second controller 222 not directly connected to the vehicle (e.g. remote/wireless controller). The second controller 222 can provide instructions for maneuvering the vehicle to the vehicle and/or the controller connected to the vehicle (e.g. adjusting the rpms of motors and the rotor blades). In some examples, a user can use the second controller 222 to maneuver the vehicle by using the visual information provided by video cameras 216. Further, in some examples, the second controller 222 can control other aspects of the vehicle (e.g. laser scanning), the other aspects being discussed further herein.

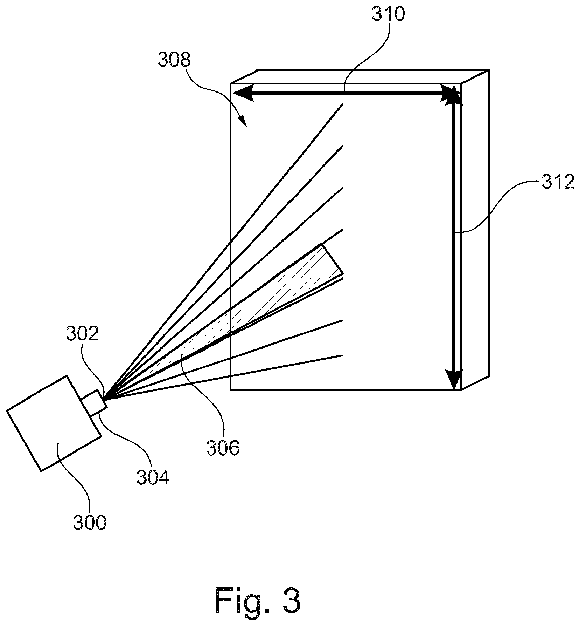

Moving to FIG. 3, FIG. 3 is a schematic view of a laser 300 comprising scanning mirrors 302 in accordance with an aspect of the invention. Scanning mirrors 302 are located in front of the beam of light emitted by laser 300 and in some examples, are integrated within the laser 300 Scanning mirrors 302 are attached to motors 304 such that when one motor rotates, a corresponding mirror attached to said motor also rotates/changes its bearing. In some examples, the scanning mirrors 302 are attached to galvanometers which can manipulate the movement of the scanning mirrors. It will be appreciated that other ways to move and/or rotate scanning mirrors 302 are contemplated such as piezoelectric actuators, or magnetostrictive actuators. Scanning mirrors 302 are used to direct a beam of light 306 emitted by laser 300 to radiate light onto a specified area of the surface 308. In the example of FIG. 3, two scanning mirrors are used such that the laser 300 can be directed to any point within a 2-dimensional grid (e.g. laterally 310 and vertically 312). In some embodiments, only one scanning mirror is used. In some examples, one or more reciprocating mirrors are used to direct the laser. Further, in some examples, other methods of directing the beam of light 306 are used such as optical lenses, prisms, deflectors, or phased array scanning. In some examples, in addition to or in lieu of scanning mirrors, a lens system is used such that the focus of the beam of light can be controlled (e.g. depth). In some such examples, a motor 304 can control the lens to change the focus of the beam of light.

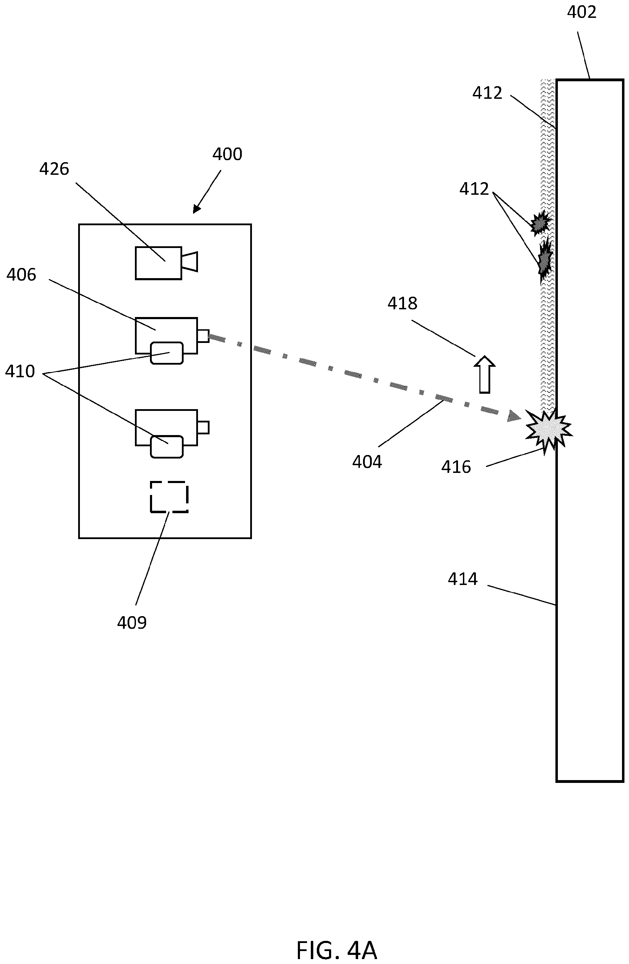

FIG. 4A is a schematic view of an example apparatus using a first laser 406 to decontaminate a surface according to an aspect of the present disclosure. In the example of FIG. 4A, the first laser 406 is an infrared (IR) laser. In some examples the first laser 406 is an ultraviolet (UV) laser as is described further herein. In the example of FIG. 4A, the IR laser 406 is emitting a beam of light 404 toward the surface 402 and is powered by a power source 409. In some examples, the power source 409 is a battery. The IR laser 406 is connected to the body of the decontamination apparatus 400 with a bracket 410. Bracket 410 can include one or more motors for moving the IR laser 406 in a desired direction. In some examples, the bracket can also include one or more gyroscopes and/or accelerometers which can direct the one or more motors to stabilize the IR laser. In some examples, the bracket 410 can direct the IR laser 406 in a desired direction and can additionally stabilize the IR laser 406 such that movement of the vehicle does not substantially change the direction of the IR laser 406. The bracket 410 of FIG. 4A can rotate the IR laser 406 to direct it in a specified direction independently of the movement of the decontamination apparatus 400. In some examples, the IR laser 406 is fixedly mounted to the decontamination apparatus 400 such that maneuvering of the decontamination apparatus effectively directs the IR laser 406 in a specific direction. In some examples, the IR laser 406 can be connected to parts of the apparatus other than the body. In some embodiments, the IR laser 406 can be removably attached to the bracket 410 such that different IR laser modules can be connected to the bracket. This can be advantageous because different applications may require different IR lasers and IR laser modules can be replaced if they break.

The IR laser 406 of FIG. 4A can emit a beam of light 404 which has wavelengths within the IR spectrum when it is energized. IR laser 406 can be any type of laser such as gas, chemical, excimer, metal vapor, solid state, fiber, photonic crystal, semiconductor, dye, free-electron laser or any combination thereof. In the example of FIG. 4A, the IR laser and can emit a beam of light 404 comprising wavelengths between 700 nanometers and 1 millimeter. In some embodiments, the IR laser is a combination of multiple lasers and in some examples, the IR laser is a laser which is frequency doubled, tripled, or the similar, to emit a beam of light comprising wavelengths between 700 nanometers and 1 millimeter. In some examples, the IR laser 406 emits a beam of light comprising of single wavelength within the IR spectrum while in other examples, the IR laser 406 emits a beam of light comprising of multiple wavelengths within the IR spectrum. The beam of light emitted from the IR laser 406 can be in the near-infrared, mid-infrared, or far-infrared electromagnetic spectrum.

By using specific portions of the IR spectrum (e.g. specific wavelengths), specific contaminants can be targeted. For example, contaminants such as bacteria can be susceptible to being destroyed when hit with a first wavelength of light, while other contaminants (e.g. viruses) are susceptible to being destroyed by a second wavelength different than the first. By using a single IR wavelength for the IR laser, a specific bacteria or virus can be targeted. In some examples, using multiple IR wavelengths for the IR laser can result in different contaminants being destroyed substantially simultaneously.

In the example of FIG. 4A, the output power and/or the intensity of the IR laser 406 is adjustable such that a desired amount of IR energy is delivered to the surface 402. By using a camera/sensor 426 which can measure the surface area and distance to the surface, a specific power and/or intensity of the IR laser beam can be utilized. This can be advantageous as the further the laser source is from the surface, the weaker the beam can be. In some examples, other sensors can be used to determine parameters which can change the effectiveness of the IR laser (e.g. atmospheric sensors detecting rain). The intensity of the IR laser 406 can be changed at any time and for any period of time. In some embodiments, the IR laser 406 operates in a continuous wave operation, whereby the output power of the laser is substantially constant over time. Alternatively, in some embodiments, the IR laser 406 operates in a pulsed operation, whereby the output power of the laser is not substantially constant over time. In some such examples, the amount of output power delivered at one time from the IR laser 406 can be higher than if the IR laser 406 operates in a continuous wave operation.

Continuing with the example of FIG. 4A, the emitted beam of light 404 from the IR laser 406 hits a surface 402 and causes the surface 402 and contaminants 412 on the surface 402 to heat up. The surface can be smooth, textured, reflective, matte, or have other surface characteristics. The contaminants 412 of FIG. 4A can include dust, germs, and/or other undesired matter on the surface. The contaminants 412 in FIG. 4 substantially heat up while portions of the surface surrounding the contaminants 412 do not heat up as much. This can be advantageous as damage to the underlying surface 402 may not be desired. The example of FIG. 4A illustrates the IR laser 406 using laser ablation to remove contaminants 412 from the surface 402. As illustrated by 416 in the example of FIG. 4A, once the contaminants 412 are sufficiently heated by the IR laser 406, the contaminants 412 evaporate, sublimate, convert to plasma, and/or are otherwise destroyed, leaving behind a decontaminated surface 414. The beam of light 404 from the IR laser can then move as indicated by 418 to perform laser ablation on the rest of the surface 402. The laser ablation process of removing contaminants can be advantageous to other forms of removing contaminants as in some examples, laser ablation does not leave a residue on the surface. Additionally, in some examples, the laser ablation process can be advantageous in that no noxious gases are released and there is no corroding effect on the surface. Further, in some examples, the laser ablation process can be faster and more precise compared to traditional forms of decontaminating the surface (e.g. water, soap, chemicals).

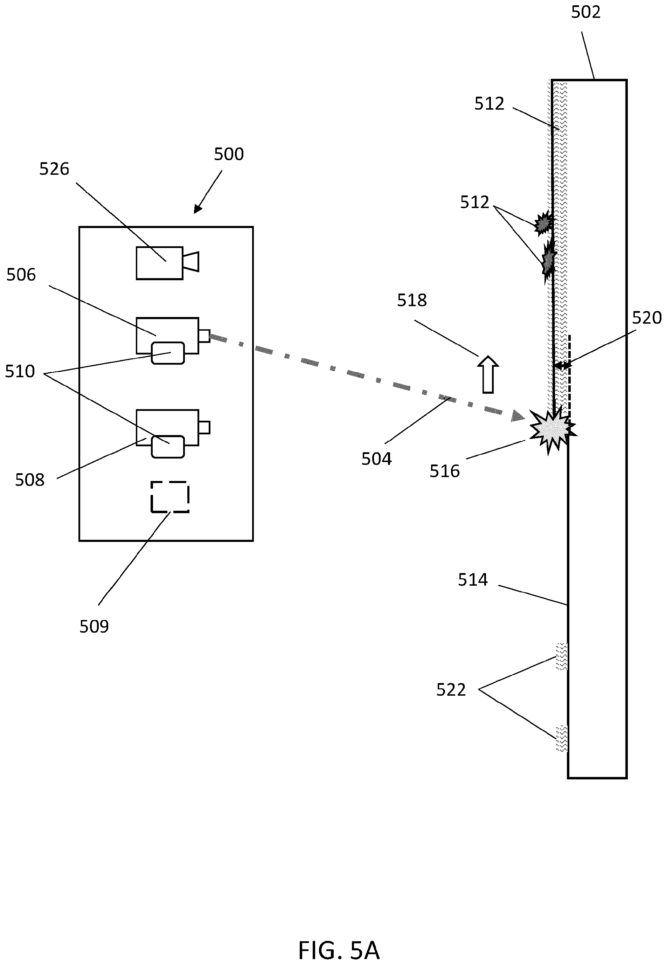

Moving to FIG. 5A, FIG. 5A illustrates the emitted beam of light 504 from the IR laser 506 hitting the surface 502, thereby heating substantially all of the surface 502. In the example of FIG. 5A, the IR laser 506 is configured to ablate a portion 520 of the entire surface 502 which includes contaminants 512. The example of ablation in FIG. 5A can be advantageous when a more thorough decontamination is required. For example, the contaminants could be of dangerous biological nature (e.g. highly contagious virus), the contaminants could be beneath other contaminants (e.g. dust/debris), and the contaminants could be contained within the surface itself. In some examples, removal of a portion of the surface 502 can be necessary to remove the contaminants. In FIG. 5A, the IR laser 506 ablates a portion 520 of the surface 502 in addition to contaminants 512 such as debris (e.g. dust). By ablating a portion 520 of the surface 502 in addition to contaminants 512 (e.g. dust), contaminants which may otherwise not have been present on the outer portion of the surface can be exposed to the outer portion of the surface. In some examples, substantially all the parts of the surface which have been decontaminated are also disinfected by the laser ablation. In some other examples, some parts of the surface which have been decontaminated are not disinfected by the laser ablation. In some examples, contaminants 522 such as germs, viruses, and other microscope organisms remain on the surface 502 after the laser ablation process. However, in some such examples, the contaminants 522 can be exposed to the top of the surface 502 after the laser ablation such that the surface 502 is suitable for further decontamination (e.g. disinfection).

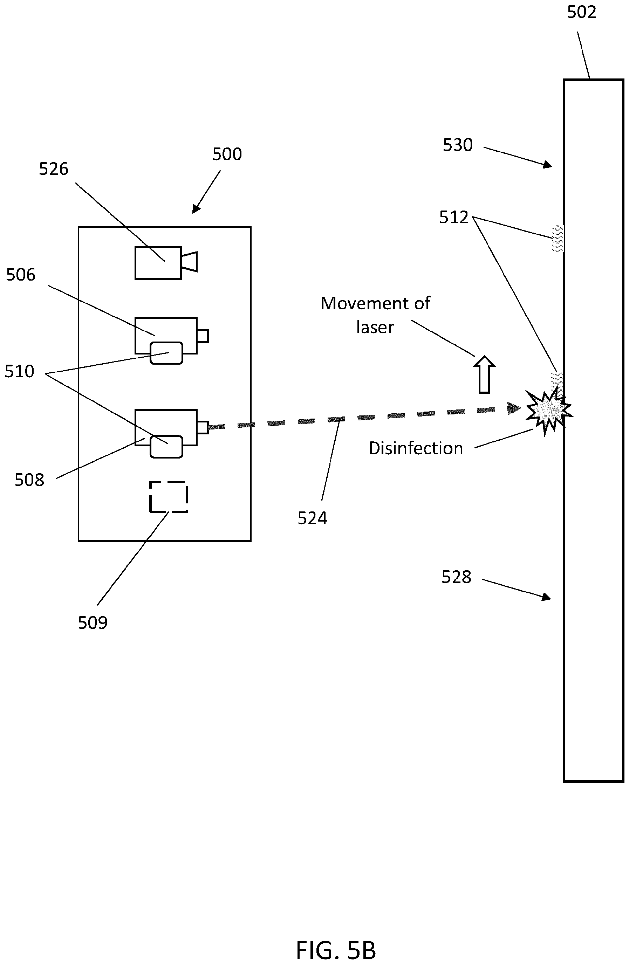

Moving to FIG. 5B, FIG. 5B is an example schematic view of the decontamination apparatus 500 in FIG. 5A comprising a second laser 508. The second laser 508 is an UV laser facing a surface 502. In some examples, the second laser can be an IR laser. In the example of FIG. 5B, the UV laser 508 is emitting a beam of light 524 toward the surface 502 and is powered by a power source 509. In some examples, the power source 509 is a battery. The UV laser 508 is connected to the body of the decontamination apparatus 500 with a bracket 510. Bracket 510 can include one or more motors for moving the UV laser 508 in a desired direction. In some examples, the bracket 510 can also include one or more gyroscopes and/or accelerometers which can direct the one or more motors to stabilize the UV laser. In some examples, the bracket 510 can direct the UV laser 508 in a desired direction and can additionally stabilize the UV laser 508 such that movement of the decontamination apparatus 500 does not substantially change the direction of the UV laser 508. The bracket 510 of FIG. 5B can rotate the UV laser 508 to direct it in a specified direction independently of the movement of the decontamination apparatus 500. In some examples, the UV laser 508 is fixedly mounted to the body of the decontamination apparatus 500 such that maneuvering of the apparatus effectively directs the UV laser 508 in a specific direction. In some examples, the UV laser 508 can be connected to parts of the apparatus other than the body. In some embodiments, the UV laser can be removably attached to the bracket such that different UV laser modules can be connected to the bracket. This can be advantageous because different applications may require different UV lasers and UV laser modules can be replaced if they break.

In some examples, the IR laser ## and the UV laser 508 are connected to independent motorized brackets such that each laser can be independently directed in a desired direction. For example, an IR laser can be directed at one location on a surface while the UV laser is directed at a different location on the surface. This can be advantageous as the lasers can operate independently of one another and perform their respective functions at the substantially the same time.

The UV laser 508 of FIG. 5B can emit a beam of light 524 which has wavelengths within the UV spectrum when it is energized. The UV laser 508 can be any type of laser such as gas, chemical, excimer, metal vapor, solid state, fiber, photonic crystal, semiconductor, dye, free-electron laser or any combination thereof. In some examples, the UV laser 508 is a monochromatic UV laser. In the example of FIG. 5B, the UV laser 508 and can emit a beam of light 524 comprising wavelengths between 10 nanometers and 450 nanometers. The beam of light 524 can be a first beam of light or a second beam of light. In some embodiments, the UV laser 508 is a combination of multiple lasers and in some examples, the UV laser 508 is a laser which is frequency doubled, tripled, or similar, to emit a beam of light comprising wavelengths between 10 nanometers and 450 nanometers. In some examples, the UV laser 508 emits a beam of light comprising of single wavelength within the UV spectrum while in other examples, the UV laser 508 emits a beam of light comprising of multiple wavelengths within the UV spectrum. The beam of light 524 emitted from the UV laser 508 can be in the near-ultraviolet, mid-ultraviolet, and/or far-ultraviolet electromagnetic spectrum. In some examples, the beam of light emitted from the UV laser can be UVA, UVB, and/or UVC rays.

By using specific portions of the UV spectrum (e.g. specific wavelengths), specific contaminants can be targeted. For example, contaminants such as bacteria can be susceptible to being destroyed by a first wavelength, while other contaminants (e.g. viruses) are susceptible to being destroyed by a second wavelength different than the first. By using a single UV wavelength for the UV laser 508, a specific bacteria or virus can be targeted. In some examples, using multiple UV wavelengths for the UV laser 508 can result in different contaminants being destroyed substantially simultaneously.

In the example of FIG. 5B, the output power and/or the intensity of the UV laser 508 is adjustable such that a desired amount of UV energy is delivered to the surface 502. By using a camera/sensor 526 which can measure the surface area and distance to the surface, a specific power and/or intensity of the UV laser beam can be utilized. This can be advantageous as the further the laser source is from the surface, the weaker the beam can be. In some examples, other sensors can be used to determine parameters which can change the effectiveness of the IR laser (e.g. atmospheric sensors detecting rain). The output power and intensity of the UV laser can be changed at any time and for any period of time. The UV laser of FIG. 5B can be advantageous compared to traditional forms of emitting UV light (e.g. UV lamps) as the intensity of emitted light from the UV laser can be constant over the surface. For examples, a UV lamp can have an intensity that is highest in the center of the area covered by UV light while having a substantially lower intensity closer to the edges of the area covered by the UV light. In contrast, the UV laser of FIG. 5B can emit a constant intensity of UV light on the surface. In some embodiments, the UV laser operates in a continuous wave operation, whereby the output power of the laser is substantially constant over time. Alternatively, in some embodiments, the UV laser operates in a pulsed operation, whereby the output power of the laser is not substantially constant over time. In some such examples, the amount of output power delivered at one time from the UV laser can be higher than an if the UV laser operates in a continuous wave operation.

Continuing with the example of FIG. 5B, the surface 502 comprises a first portion 528 and second portion 530, wherein the first portion 528 has already been exposed to the beam of light 524 from the UV laser 508 while the second portion 530 has yet to be exposed to the beam of light 524 from the UV laser 508. The emitted beam of light 524 from the UV laser 508 hits the surface 502 and contaminants on the surface 512 (e.g. germs). The beam of UV light 524 is then absorbed by the contaminants 512 and can cause microorganisms such as bacteria, viruses, and fungi to be killed or inactivated. The beam of UV light 524 can destroy the nucleic acids of the microorganisms such that their DNA is disrupted, thereby causing the microorganisms to no longer be able to perform cellular functions (e.g. reproduction) and causing the microorganisms to be destroyed. In some examples, the use of UV light to destroy microorganisms can be considered disinfection. In the examples of FIG. 5B, the first portion 528 of the surface 502 which has been exposed to the beam of light 524 from the UV laser 508 illustrates that the UV radiation can cause substantially all microorganisms to be destroyed. However, in some examples, only a portion of the microorganisms on the surface are destroyed. The UV laser can be advantageous to other methods of disinfection (e.g. chemical disinfection) as it can be used on surfaces which may otherwise be harmed by using chemicals, water, or soap. Additionally, in some examples, the UV laser can be advantageous to disinfect surfaces as it does not leave a residue on the surface. In some embodiments, the UV laser does not release noxious gases and does not corrode the surface that is being disinfected. Further, in some examples, the UV laser can be quicker and more precise compared to other forms of disinfecting the surface (e.g. chemicals, UV lamps, soap).

As illustrated by FIG. 5A and FIG. 5B, a first laser 506 and a second laser 508 can be used to decontaminate the surface 502. In the example of FIG. 5A and FIG. 5B, the first laser 506 is an IR laser and the second laser 508 is a UV laser. In some examples, the first laser is a UV laser and the second laser is an IR laser, and in some examples, both lasers are IR lasers or UV lasers. It will be appreciated that other combinations of IR and UV lasers are contemplated. It can be advantageous to have the first laser be an IR laser and the second laser be an UV laser as the IR laser can expose contaminants which would otherwise not be exposed using IR laser ablation. For example, in FIG. 5A, a portion of the surface is ablated by the IR laser 506 along with contaminants such as dust, thereby exposing germ contaminants which are located within the surface or behind the dust. Removing these contaminants can allow the UV laser 508 to be more effective as fewer contaminants (e.g. germs) are blocked from being hit by the beam of light emitted by the UV laser. Additionally, using the IR laser first can be advantageous as in some examples, substantially all contaminants are removed from the surface without requiring the UV laser to be used on the surface. However, in some examples, it can be advantageous to use the UV laser first as the UV laser can require lower power than the IR laser. In some examples, using the UV laser first can be advantageous as the surface may have already been substantially decontaminated, thus using the IR laser may not be required.

Further, in some examples, both the IR laser and UV laser can be active at substantially the same time. In some such examples, the IR laser can pass over a surface before the UV laser passes over the surface. One advantage of having both the IR laser and the UV laser active substantially at the same time can be that the time to decontaminate multiple surfaces can be decreased.

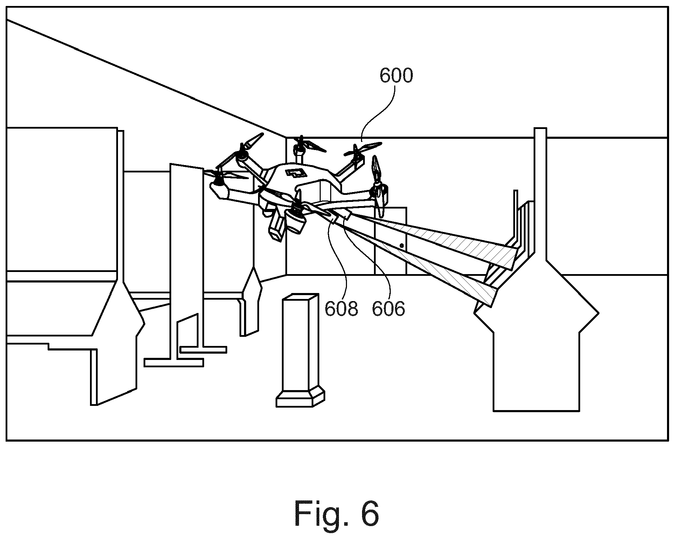

Referring now to FIG. 6, FIG. 6 is a perspective view of an example aerial vehicle 600 with an IR laser 606 and UV laser 608 decontaminating an indoor area according to an aspect of the present disclosure. The IR laser 606 and the UV laser 608 are attached to the aerial vehicle 600 such that they can be directed independently of movement from the aerial vehicle. The configuration of the IR laser and UV laser attached to the aerial vehicle can be advantageous as the aerial vehicle can move more freely than other vehicles (e.g. ground-based and/or water-based). One advantage of using an aerial vehicle for decontamination using an IR laser and UV laser is that the aerial vehicle can reposition the IR laser or UV laser to better face a surface. For example, a UV laser can be less effective if the beam of light it emits hits the surface at an angle other than substantially orthogonal. In some such examples, the aerial vehicle can reposition the UV laser such that the beam of light it emits hit the surface at a substantially orthogonal angle. Another advantage of using an aerial vehicle in comparison to other vehicles is that it can have superior maneuverability. For example, a ground-based vehicle could have difficulty reaching areas that are high without additional hardware. In another example, a ground-based vehicle could have difficulty maneuvering in small spaces such as the inside of a larger vehicle (e.g. bus).

The aerial vehicle 600 of FIG. 6 is an unmanned, remote-controlled aerial vehicle. In some examples, the aerial vehicle can be a manned aerial vehicle wherein an operator within the aerial vehicle controls aspects of the vehicle. One advantage of an unmanned, remote-controlled aerial vehicle can be the remote operation of the aerial vehicle. For example, the remote-controlled vehicle can decontaminate surfaces without exposing the operator to the contaminants on the surface. The contaminants can be of a biological nature which could harm an operator that came in contact with the contaminants. By being remote-controlled, operator exposure to the contaminants can be avoided.

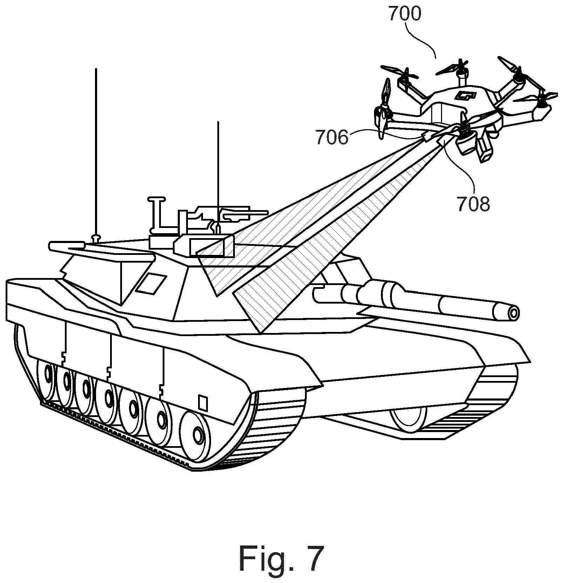

Referring now to FIG. 7, FIG. 7 is a perspective view of an aerial vehicle ## comprising an IR laser and an UV laser according to an aspect of the present disclosure. In the example of FIG. 7, the aerial vehicle can use IR laser 706 and UV laser 708 to decontaminate surfaces of an object outdoors. The IR laser 706 and UV laser 708 emit beams of light at the surfaces of the object as previously described herein. In some examples, the IR laser 706 is used primarily in outdoor environments as it can have a higher output power than the UV laser 708.

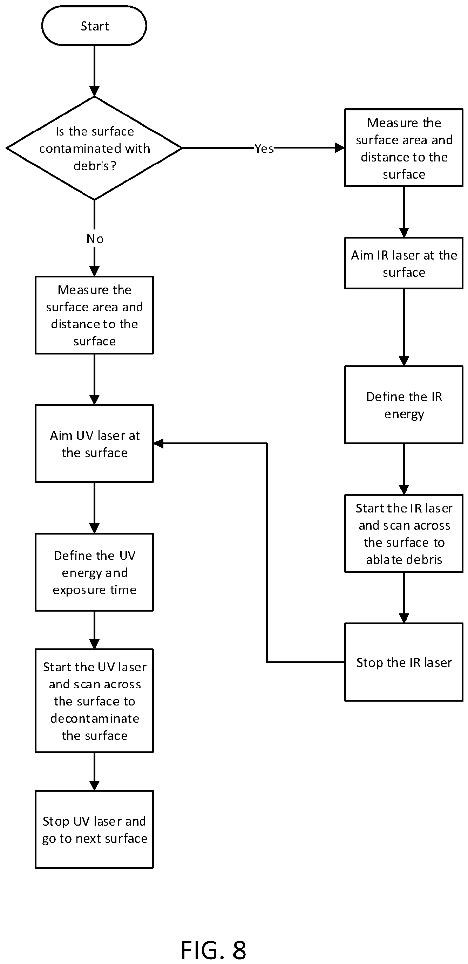

Moving to FIG. 8, FIG. 8 is an example flow diagram of decontaminating a surface process according to an aspect of the present disclosure. In the example process of FIG. 8, the first step is to determine if the surface to be decontaminated is currently contaminated with dust and/or other debris. In some examples, this is done by using video cameras which can capture an image and a processor can compare the image to a previous image to determine any changes (e.g. debris contamination). In some examples, captured images can be used to determine other characteristics of the surface such as a color change which may indicate contaminants are present. In further examples, ultrasonic sensors can be used to determine if the surface is contaminated and how much contamination is present. Other methods for determining if the surface is contaminated are contemplated. If the surface is contaminated with dust and/or debris which might decrease the effectiveness of a UV laser, the decontamination apparatus can measure the surface area and the distance to the surface in preparation for decontamination using an IR laser. The IR laser is then aimed at the surface. Continuing with the method in FIG. 8, the IR energy is then defined (e.g. deciding to use a desired power for a desired amount of time). By defining the IR energy which will be used, the IR laser can be configured to sufficiently decontaminate a surface to a desired degree. For example, the IR laser can be configured to decontaminate the surface without damaging the under lying surface or in some examples, the IR laser can ablate a specific portion of the surface. Once the IR energy is defined, the IR laser is started such that it emits a beam of light onto the surface. The IR laser is then scanned across the surface laterally and longitudinally, decontaminating, ablating debris and/or a portion of the surface in the process. The amount of time the IR laser is used on the surface can be varied and the IR laser can be turned on and off during the process of decontamination. Once the surface has been decontaminated by the IR laser, the IR laser is then stopped. Continuing with the decontamination process, the UV laser is then aimed at the surface. While measuring the surface area and distance to the surface is not required, due to this step being done before aiming the IR laser, in some examples, it can be advantageous to measure again. For example, the surface area and distance to the surface may need to be remeasured if the decontamination apparatus moves before aiming the UV laser at the surface. Once the UV laser is aimed at the surface, the UV energy and exposure time is defined. By defining the UV energy and exposure time, the UV laser can be configured to sufficiently decontaminate a surface to a desired degree. For example, the UV laser can be configured to disinfect or sterilize the surface. After the UV energy and exposure time have been defined, the UV laser is then scanned across the surface laterally and longitudinally, decontaminating the surface in the process. After the surface has been decontaminated, the UV laser is stopped, and the decontamination apparatus can go to the next surface. However, if the surface is not contaminated with dust or other debris which might interfere with the UV laser, only the UV laser is used to decontaminate the surface in accordance with the steps described above.

The processes of decontamination using the lasers can continue until an entire predetermined space is decontaminated. In some examples, the predetermined space is substantially all exposed surfaces of the space while in some examples, portions of the space are not covered by the decontamination process. This can be advantageous as some portions of a space can require a different method of decontamination.

## Claims

1. An apparatus (100, 104, 200, 600, 700) for decontaminating surfaces (308, 402, 502) comprising:
• a body (202) comprising one or more motors (204) for maneuvering the body;
• a power source (210) supported by the body;
• a first laser (106, 206, 606, 706) supported by the body and powered by the power source, the first laser configured to emit a first beam of light, the first beam of light comprising wavelengths between 700 nanometers and 1 millimeter;
• a second laser (108, 208, 608, 708) supported by the body and powered by the power source, the second laser configured to emit a second beam of light, the second beam of light comprising wavelengths between 10 nanometers and 450 nanometers;
• the first laser and the second laser able to remove contaminants from a surface by emitting the first beam of light and the second beam of light onto the surface (308, 402, 502).

2. The apparatus of claim 1, further comprising one or more video cameras (216) mounted to the body (202) and configured to help maneuver the apparatus.

3. The apparatus of any of the preceding claims, further comprising one or more video cameras (216) mounted to the body (202) and configured to identify a target to be irradiated.

4. The apparatus of any of the preceding claims, further comprising a global positioning system, or in short GPS, sensor (218) and an altitude sensor (220).

5. The apparatus of any of the preceding claims, wherein the first laser (106, 206, 606, 706) is a pulsed infrared, in short IR, laser (406, 506).

6. The apparatus of any of the preceding claims, wherein the second laser is a monochromatic ultraviolet, in short UV, laser (508).

7. The apparatus of any of the preceding claims, further comprising a controller (214), the controller configured to maneuver the apparatus by controlling the one or more motors (204), the controller further configured to control operations of the first laser (106, 206, 606, 706) and the second laser (208).

8. The apparatus of claim 7, further comprising a wireless control (222), wherein the wireless control (222) is connected to the controller (214) and is configured to provide remote control over the controller (214).

9. The apparatus of claim 7 or 8, wherein the controller (214) is configured such that the apparatus (100, 104, 200, 600, 700) maneuvers in a predetermined path.

10. A method of decontaminating a surface (308, 402, 502) comprising:
• determining if a surface (308, 402, 502) is contaminated with debris;
• measuring a surface area and a distance to the surface (308, 402, 502);
• aiming an IR laser at the surface (308, 402, 502);
• defining an amount of IR energy to be used;
• scanning the IR laser across the surface, the IR laser configured to emit a first beam of light, the first beam of light comprising wavelengths between 700 nanometers and 1 millimeter;
• aiming a UV laser at the surface;
• defining an amount of UV energy to be used; and
• scanning the UV laser across the surface, the UV laser configured to emit a second beam of light, the second beam of light comprising wavelengths between 10 nanometers and 450 nanometers and thereby decontaminating the surface (308, 402, 502).

11. The method of claim 10, wherein a user directs a decontamination device (100, 102, 104, 200, 600, 700) to perform the method remotely.

12. The method of claim 10 or 11, wherein scanning the IR laser across the surface (308, 402, 502) further comprises controlling the first beam of light across the surface (308, 402, 502) in a predetermined pattern such that the first beam of light hits substantially all portions of the surface (308, 402, 502).

13. The method of any of the claims 10 - 12, wherein scanning the UV laser across the surface (308, 402, 502) further comprises controlling the second beam of light across the surface in a predetermined pattern such that the second beam of light hits substantially all portions of the surface (308, 402, 502).

14. A method of decontaminating a surface (308, 402, 502) comprising:
• maneuvering a decontamination apparatus (100, 104, 200, 600, 700) to a position, the position being a distance from a surface, the decontamination apparatus comprising a first laser (106, 206, 606, 706) and a second laser, wherein the first laser (106, 206, 606, 706) is configured to emit a first beam of light, the first beam of light comprising wavelengths between 700 nanometers and 1 millimeter, and the second laser is configured to emit a second beam of light, the second beam of light comprising wavelengths between 10 nanometers and 450 nanometers;
• measuring a surface area and/or the distance to the surface (308, 402, 502);
• aiming the first laser (106, 206, 606, 706) and/or the second laser (108, 208, 608, 708) at the surface;
• energizing the first laser and/or the second laser such that it emits a beam of light toward the surface (308, 402, 502);
• scanning the energized first laser and/or the second laser across the surface (308, 402, 502), thereby decontaminating the surface (308, 402, 502).

15. An apparatus for decontaminating a surface comprising:
• a body;
• a first laser (106, 206, 606, 706) supported by the body and configured to emit a first beam of light, the first beam of light comprising wavelengths between 700 nanometers and 1 millimeter;
• a second laser (108, 208, 608, 708) supported by the body and configured to emit a second beam of light, the second beam of light comprising wavelengths between 10 nanometers and 450 nanometers;
• the first laser and the second laser able to remove contaminants from a surface (308, 402, 502) by emitting the first beam of light and the second beam of light onto or toward the surface (308, 402, 502) in order to decontaminate the surface (308, 402, 502).

16. The apparatus of claim 15, further comprising a power source supported by the body and whereby the body further has one or more supports for supporting the body above a ground surface (110).

17. The apparatus of on of the claims 15 or 16, further comprising one or more scanning mirrors (302), the one or more scanning mirrors (302) configured to change the bearing of at least one of the first laser (106, 206, 606, 706) and the second laser (108, 208, 608, 708

18. The apparatus of claim 17 or any of the preceding claims, wherein the first beam of light hits the surface (308, 402, 502) at a time before the second beam of light hits the surface (308, 402, 502).

19. A method for decontaminating a surface (308, 402, 502) comprising:
• aiming an IR laser at a surface (308, 402, 502), the IR laser configured to emit a first beam of light, the first beam of light comprising wavelengths between 700 nanometers and 1 millimeter;
• energizing the IR laser such that the first beam of light hits the surface;
• aiming a UV laser at the surface (308, 402, 502), the UV laser configured to emit a second beam of light, the second beam of light comprising wavelengths between 10 nanometers and 450 nanometers;
• energizing the UV laser such that the second beam of light hits the surface (308, 402, 502);
• the first beam of light and the second beam of light thereby decontaminating the surface (308, 402, 502).

20. The method of claim 19, further comprising scanning the IR laser and the UV laser across the surface (308, 402, 502),.
